(19) Europäisches Patentamt / European Patent Office / Office européen des brevets

(11) **EP 4 152 000 A1**

(12) **EUROPEAN PATENT APPLICATION**
published in accordance with Art. 153(4) EPC

(43) Date of publication:
**22.03.2023 Bulletin 2023/12**

(21) Application number: **21832401.0**

(22) Date of filing: **29.06.2021**

(51) International Patent Classification (IPC):
**G01N 33/49** (2006.01)      **G01N 33/50** (2006.01)
**G01N 33/68** (2006.01)

(52) Cooperative Patent Classification (CPC):
**G01N 33/49; G01N 33/50; G01N 33/68**

(86) International application number:
**PCT/JP2021/024579**

(87) International publication number:
**WO 2022/004730 (06.01.2022 Gazette 2022/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.06.2020 JP 2020113607**

(71) Applicant: HORIBA, Ltd.
**Kyoto-shi, Kyoto 601-8510 (JP)**

(72) Inventors:
• SAITO, Kensuke
  **Kyoto-shi, Kyoto 601-8510 (JP)**
• YOSHIOKA, Kazunori
  **Kyoto-shi, Kyoto 601-8510 (JP)**

(74) Representative: **Isarpatent
Patent- und Rechtsanwälte Barth
Charles Hassa Peckmann & Partner mbB
Friedrichstrasse 31
80801 München (DE)**

(54) **BODY FLUID ANALYSIS DEVICE, METHOD FOR DETERMINING BODY FLUID SAMPLE, AND COMPUTER PROGRAM**

(57) The present invention provides a body fluid analysis device, a determination method, and a computer program that can afford determination information assisting differentiation of patients (viral infection/bacterial infection, or severity of viral infection). The device has a parameter receiving part 600 that receives plural kinds of parameters used for the determination of (viral infection/bacterial infection, or severity of viral infection), and an infection determining part 700 configured to calculate information of the determination based on the plural kinds of parameters. The infection determining part is configured to calculate information of the determination by using the relational expression (I) stored in advance and based on the plural kinds of parameters. The plural kinds of parameters include at least CRP value, and the relational expression (I) is a multiple regression equation or multiple discriminant equation, determined in advance by multivariate analysis of the past data. The device can perform the determination method of the present invention and execute the computer program of the present invention.

[Fig. 2]

```
                    start
                      |
        +-------------+-------------+
        |                           |
s1: sample liquid for CRP     s5: sample liquid for white
measurement is formed in      blood cell counting is
    CRP chamber               formed in WBC chamber
        |                           |
s2: measured value            s6: white blood cells are
corresponding to CRP value    counted in WBC chamber
is obtained in CRP chamber           |
        |                           |
s3: measured value            s7: measured value receiving
receiving part receives CRP   part receives measured value
    parameter value           of white blood cell counting
        |                           |
s4: CRP value calculating     s8: white blood cell count
part determines CRP value     calculating part calculates
        |                         white blood cell count
        |                           |
        +-------------+-------------+
                      |
s9: infection determining part substitutes CRP value and
white blood cell count into relational expression (I) stored
as explanatory variables relating to test subject and
calculates objective variable Y and discriminant information
of whether viral infection or bacterial infection
                      |
                     end
```

EP 4 152 000 A1

**Description**

[Technical Field]

The present invention relates to a body fluid analysis device that assists physicians in the differentiation of whether a patient is suffering from a viral infection or a bacterial infection, or differentiation of the severity of a patient with a viral infection (e.g., COVID-19), and provides information that assists physicians in sufficiently fulfilling their obligation to explain medical condition to the patient or the patient's guardian. In addition, the present invention relates to a method for determining whether a body fluid sample collected from a test subject is obtained from a test subject with a viral infection or from a test subject with a bacterial infection, or whether a body fluid sample collected from a patient with a viral infection was obtained from a severely ill patient or a mildly ill patient, and a computer program for the determination.

[Background Art]

Conventionally, when a physician diagnoses a patient complaining of symptoms such as a cold and the like and prescribes a drug, the physician differentiates whether the patient is suffering from a viral infection or a bacterial infection based on the results of examinations by interviews and auscultations, and increase and decrease in the numerical values of each test item included in the clinical test (especially blood test) results, and prescribes drugs in consideration of the differential results. In the following, "differentiates whether the patient is suffering from a viral infection or a bacterial infection" is also simply referred to as "differentiation".

When prescribing drugs, physicians are obliged to sufficiently explain to the patient (or the patient's guardian) the diagnosis results (disease name, medical condition, etc.), details of the drug to be prescribed, side effects of the drug, and the like. In recent years, as the drugs that can be used for treatment, and the like diversify, it is necessary to provide such information to patients, etc. in a form that is easier for them to understand.

In addition, one of the purposes of considering the results of differentiation when prescribing drugs is to determine whether antibiotics (also including antibacterial drugs) should be administered. This is because the administration of antibiotics may be not only ineffective in treating viral infections, but also may cause new problems such as a decrease in indigenous bacteria and the development of resistant bacteria.

On the other hand, it is conventionally known that blood test values such as white blood cell (hereinafter sometimes to be referred to as "WBC") count, C reactive protein (hereinafter sometimes to be referred to as "CRP") value, and the like can be referred to as indicators for differentiation (e.g., Non Patent Literature 1).

[Citation List]

[Patent Literature]

[PTL 1]
JP-A-11-108923
[PTL 2]
JP-A-2014-215210

[Non Patent Literature]

[NPL 1]
Takafumi Okada et al., "A practical approach estimating etiologic agents using real-time PCR in pediatric inpatients with community-acquired pneumonia", J Infect Chemother (2012) 18:832-840

[Summary of Invention]

[Technical Problem]

However, in the above-mentioned Non Patent Literature 1, the relationships between the measured values (test values) of individual blood test items such as white blood cell count and CRP value, and viral infection and bacterial infection are individually shown (for each item). Therefore, when a physician tries differentiation from a patient's blood test results based on such findings, the final differentiation will be made by logically considering together the test values of individual test items: for example, (Because the white blood cell count belongs to the region of viral infection and the CRP value also belongs to the region of viral infection, it is differentiated as a viral infection).

In the above-mentioned differentiation process, contents such as what test values should be combined to achieve effective differentiation and which test value is given priority when combining multiple test values rely on the ability of individual physicians and erroneous differential results may be provided. As a result, as mentioned above, wrong prescription may be made by prescribing antibiotics for a viral cold, which in turn may cause new problems such as the emergence of resistant bacteria and the like.

Separately therefrom, medical shortages due to the recent spread of the infection with novel coronavirus (SARS-CoV-2) are feared. Even if positive in the PCR test, one may be forced to wait at home or accommodation in the absence of subjective symptoms or on judgment of a mild case based on the complaints, as a result of which there have been reports of cases in which the disease aggravated rapidly to cause death without any medical treatments. Therefore, a method that can objectively determine the severity of or the risk of aggravation in patients with viral infection including COVID-19 is demanded.

The problem of the present invention is to solve the above-mentioned problems, and provide a body fluid analysis device that provides determination information useful for physicians, etc. in differentiating whether a patient is suffering from a viral infection or a bacterial infection, or whether a patient with a viral infection is in a severe condition, a method capable of providing the determination information, and a computer program capable of providing the determination information. The body fluid analysis device, the method, and the computer program are suitable for making a confirmed diagnosis by a physician as to whether a patient is suffering from a viral infection or a bacterial infection, supporting (assisting) a public health center, etc. in determining whether or not a patient with a viral infection needs to be hospitalized, and obtaining information that is easier for patients (or patient's guardian) to understand in fulfilling the physicians' obligation to explain.

[Solution to Problem]

The main constitution of the present invention includes the following.

[1] A body fluid analysis device, comprising

a parameter receiving part that receives plural kinds of parameters for use in the determination of whether a test subject is suffering from a viral infection or a bacterial infection, wherein the aforementioned plural kinds of parameters comprise a CRP value of a body fluid sample collected from the test subject, and test value(s) of one or more body fluid test items selected from body fluid test items other than the CRP value, and an infection determining part configured to calculate determination information of whether the aforementioned test subject is suffering from a viral infection or a bacterial infection based on the aforementioned plural kinds of parameters and using a relational expression (I) described below and stored in advance, wherein the aforementioned relational expression (I) is a formula showing a relationship between explanatory variables and an objective variable as determined by a multivariate analysis,

wherein the explanatory variables are the same plural kinds of parameters as the aforementioned plural kinds of parameters and obtained from a plurality of data providers known to have suffered from a viral infection and a plurality of data providers known to have suffered from a bacterial infection, and the objective variable is determination information as to whether each of the aforementioned plurality of data providers suffered from a viral infection or a bacterial infection.

[1a] A body fluid analysis device, comprising

a parameter receiving part that receives plural kinds of parameters for use in the determination of whether a patient with a viral infection is severely ill, wherein the aforementioned plural kinds of parameters comprise a CRP value of a body fluid sample collected from the test subject, and test value(s) of one or more body fluid test items selected from body fluid test items other than the CRP value, and an infection determining part configured to calculate determination information of whether the aforementioned patient with the viral infection is severely ill based on the aforementioned plural kinds of parameters and using a relational expression (I) described below and stored in advance, wherein the aforementioned relational expression (I) is a formula showing a relationship between explanatory variables and an objective variable as determined by a multivariate analysis, wherein the explanatory variables are the same plural kinds of parameters as the aforementioned plural kinds of parameters and obtained from a plurality of data providers known to have suffered from a severe viral infection and a plurality of data providers known to have suffered from the same but non-severe bacterial infection, and the objective variable is determination information as to whether each of the aforementioned plurality of data providers suffered from a severe viral infection or the same but non-severe viral infection.

[2] The body fluid analysis device of the above-mentioned [1], wherein the above-mentioned multivariate analysis comprises one or more analyses selected from a discriminant analysis, a logistic regression analysis, a multiple regression analysis, a Cox hazard regression analysis, a principal component analysis, a factor analysis, a variance analysis, and a cluster analysis.

[2a] The body fluid analysis device of the above-mentioned [1a], wherein the above-mentioned multivariate analysis comprises one or more analyses selected from a discriminant analysis, a logistic regression analysis, a multiple regression analysis, a Cox hazard regression analysis, a principal component analysis, a factor analysis, a variance analysis, and a cluster analysis.

[3] The body fluid analysis device of the above-mentioned [1] or [2], wherein the above-mentioned test value(s) of the body fluid sample relating to the one or more body fluid test items selected from body fluid test items other than the CRP value comprise(s) a white blood cell count, and the body fluid analysis device further comprises a white blood cell measuring chamber and a white blood cell count calculating part for measuring the white blood cell count of the body fluid sample collected from the above-mentioned test subject, and a CRP measuring chamber and a CRP value calculating part for measuring the CRP value of the body fluid sample.

[3a] The body fluid analysis device of the above-mentioned [1a] or [2a], wherein the above-mentioned test value(s) of the body fluid sample relating to the one or more body fluid test items selected from body fluid test items other than the CRP value comprise(s) a white blood cell count, and the body fluid analysis device further comprises a white blood cell measuring chamber and a white blood cell count calculating part for measuring the white blood cell count of the body fluid sample collected from the above-mentioned test subject, and a CRP measuring chamber and a CRP value calculating part for measuring the CRP value of the body fluid sample.

[4] The body fluid analysis device of any of the above-mentioned [1] to [3], wherein the plural kinds of parameters for use in the determination of whether the above-mentioned test subject is suffering from a viral infection or a bacterial infection comprise a neutrophil count, and the above-mentioned infection determining part is configured to calculate determination information showing the possibility of the above-mentioned test subject suffering from a viral infection or a bacterial infection based on at least the white blood cell count and the CRP value and/or at least the neutrophil count and the CRP value included in the aforementioned test values, and using the above-mentioned relational expression (I).

[4a] The body fluid analysis device of any of the above-mentioned [1a] to [3a], wherein the above-mentioned plural kinds of parameters for use in the determination of whether a patient with the viral infection is severely ill comprise a platelet count, and the above-mentioned infection determining part is configured to calculate determination information showing whether the above-mentioned patient with the viral infection is severely ill based on at least the platelet count and the CRP value and/or at least the platelet count, the white blood cell count, and the CRP value included in the aforementioned test values, and using the above-mentioned relational expression (I).

[5] The body fluid analysis device of any of the above-mentioned [1] to [4], further comprising a chamber and a neutrophil count calculating part for measuring a neutrophil count of the body fluid sample collected from the above-mentioned test subject.

[5a] The body fluid analysis device of any of the above-mentioned [1a] to [4a], further comprising a chamber and a platelet count calculating part for measuring a platelet count of the body fluid sample collected from the above-mentioned patient with a viral infection.

[6] The body fluid analysis device of any of the above-mentioned [1] to [5], wherein the body fluid is blood.

[6a] The body fluid analysis device of any of the above-mentioned [1a] to [5a], wherein the body fluid is blood.

[7] A method for determining whether a body fluid sample derived from a test subject is obtained from a test subject with a viral infection or a test subject with a bacterial infection, comprising

a first step of preparing plural kinds of parameters (A) below, and
a second step of determining whether the aforementioned body fluid sample is obtained from a test subject with a viral infection or a test subject with a bacterial infection by using the aforementioned plural kinds of parameters and the following relational expression (I), wherein the plural kinds of parameters (A) comprise a CRP value of the aforementioned body fluid sample and test value(s) of one or more body fluid test items selected from body fluid test items other than the CRP value, and wherein the relational expression (I) is a formula showing a relationship between explanatory variables and an objective variable as determined by a multivariate analysis, wherein the explanatory variables are the same plural kinds of parameters as said plural kinds of parameters and obtained from a plurality of data providers known to have suffered
from a viral infection and a plurality of data providers known to have suffered from a bacterial infection, and the objective variable is determination information as to whether each of said plurality of data providers suffered from a viral infection or a bacterial infection.

[7a] A method for determining whether a body fluid sample derived from a test subject is obtained from a test subject suffering from a severe viral infection or a test subject suffering from the same but non-severe viral infection, comprising

a first step of preparing plural kinds of parameters of the following (A), and
a second step of determining whether the aforementioned body fluid sample is obtained from a test subject suffering from a severe viral infection or a test subject suffering from the same but non-severe viral infection by using the aforementioned plural kinds of parameters and the following relational expression (I), wherein the plural kinds of parameters of (A) comprise a CRP value of the aforementioned body fluid sample and test value(s)of one or more body fluid test items selected from body fluid test items other than the CRP value, and wherein the relational expression (I) is a formula showing a relationship between explanatory variables and an objective variable as determined by a multivariate analysis,
wherein the explanatory variables are the same plural kinds of parameters as the aforementioned plural kinds of parameters and obtained from a plurality of data providers known to have suffered from a severe viral infection and a plurality of data providers known to have suffered from the same but non-severe bacterial infection, and the objective variable is determination information as to whether each of the aforementioned plurality of data providers suffered from a severe viral infection or the same but non-severe viral infection.

[8] The method of the above-mentioned [7], wherein the above-mentioned test value(s)of the body fluid sample relating to the one or more body fluid test items selected from body fluid test items other than the CRP value in the plural kinds of parameters of the above-mentioned (A) comprises a white blood cell count.

[8a] The method of the above-mentioned [7a], wherein the above-mentioned test value(s)of the body fluid sample relating to the one or more body fluid test items selected from body fluid test items other than the CRP value in the plural kinds of parameters of the above-mentioned (A) comprises a white blood cell count and/or a platelet count.

[9] The method of the above-mentioned [7] or [8], wherein the body fluid is blood.

[9a] The method of the above-mentioned [7a] or [8a], wherein the body fluid is blood.

[10] A computer program for functioning a computer as a parameter receiving means that receives plural kinds of parameters of the following (A) for use in the determination of whether a test subject is suffering from a viral infection or a bacterial infection, and as an infection determining means that calculates determination information of whether the aforementioned test subject is suffering from a viral infection or a bacterial infection based on the aforementioned plural kinds of parameters and using the following relational expression (I),

wherein the plural kinds of parameters of (A) comprise a CRP value of the aforementioned body fluid sample derived from the test subject and test value(s) of one or more body fluid test items selected from body fluid test items other than the CRP value, and wherein the relational expression (I) is a formula showing a relationship between explanatory variables and an objective variable as determined by a multivariate analysis,
wherein the explanatory variables are the same plural kinds of parameters as said plural kinds of parameters and obtained from a plurality of data providers known to have suffered from a viral infection and a plurality of data providers known to have suffered from a bacterial infection, and the objective variable is determination information as to whether each of said plurality of data providers suffered from a viral infection or a bacterial infection.

[10a] A computer program for functioning a computer as a parameter receiving means that receives plural kinds of parameters of the following (A) for use in the determination of whether a patient with a viral infection is severely ill, and as an infection determining means that calculates determination information of whether the aforementioned patient with a viral infection is severely ill based on the aforementioned plural kinds of parameters and using the following relational expression (I),
wherein the plural kinds of parameters of (A) comprise a CRP value of a body fluid sample derived from the aforementioned patient with a viral infection and test value(s) of one or more body fluid test items selected from body fluid test items other than the CRP value, and wherein the relational expression (I) is a formula showing a relationship between explanatory variables and an objective variable as determined by a multivariate analysis,
wherein the explanatory variables are the same plural kinds of parameters as the aforementioned plural kinds of parameters and obtained from a plurality of data providers known to have suffered from a severe viral infection and a plurality of data providers known to have suffered from the same but non-severe bacterial infection, and the objective variable is determination information as to whether each of the aforementioned plurality of data providers suffered from a severe viral infection or the same but non-severe viral infection.

[11] The computer program of the above-mentioned [10], wherein the above-mentioned test value(s)of the body fluid sample relating to the one or more body fluid test items selected from body fluid test items other than the CRP value in the plural kinds of parameters of the above-mentioned (A) comprises a white blood cell count.

[11a] The computer program of the above-mentioned [8a], wherein the above-mentioned test value(s)of the body fluid sample relating to the one or more body fluid test items selected from body fluid test items other than the CRP value in the plural kinds of parameters of the above-mentioned (A) comprises a white blood cell count and/or a platelet count.

[12] The method of the above-mentioned [10] or [11], wherein the body fluid is blood.

[12a] The method of the above-mentioned [10a] or [11a], wherein the body fluid is blood.

[Advantageous Effects of Invention]

Non Patent Literature 1 separately clarifies the relationship between individual items of blood tests such as white blood cell count, CRP value, and the like, and viral infection, as well as bacterial infection. That is, in Non Patent Literature 1, like the relationship between viral infection and white blood cell counts, and the relationship between bacterial infection and white blood cell counts, the test values of individual blood test items are linked to viral infection or bacterial infection, and the variations in the white blood cell count and CRP value are shown separately based on the idea of single regression analysis.

In contrast, in the present invention, test values of a plurality of items such as white blood cell count/neutrophil count, CRP value, and the like are used as explanatory variables for a multivariate analysis, and are associated with each other to determine viral infection or bacterial infection (objective variable). This increases the possibility of obtaining more correct differentiation results than when associating each test value independently with differentiation. In the Examples described later, the area under the ROC curve analyzed multivariately (hereinafter sometimes to be abbreviated as "AUC") is a numerical value extremely close to 1, and it is understood that the plural kinds of parameters employed in the present invention provide highly accurate differentiation results. As described in detail in the below-mentioned Example, the multivariate analysis method and calculation formula used in the present invention were achieved by processing 783 samples for which confirmed diagnosis of viral infection (RSV subtype A & B, Parainfluenza virus 1 & 2, Parainfluenza virus 3 & Human metapneumovirus, Influenza virus A & B, and Rhinovirus & Enterovirus (optional)) or bacterial infection (S. pneumoniae, H. influenzae, M. pneumoniae, C. pneumoniae, S. pyogenes, and L. pneumophila) was made using the results of real-time PCR. Therefore, the method and the like are very valid and can achieve the desired differentiation with very superior accuracy.

Therefore, physicians can refer to the determination information indicated by the body fluid analysis device and differentiate viral infection and bacterial infection with higher credibility. As a result, erroneous administration of antibiotics can be reduced, and the reduction of indigenous bacteria and the development of resistant bacteria can also be suppressed. Furthermore, as will be understood from the results of AUC in the below-mentioned Examples, the differentiation information obtained from the device of the present invention is extremely reliable, and the display format of the information permits instantaneous recognition of having a viral infection or a bacterial infection. Therefore, physicians can use the information obtained from the device of the present invention to fulfill the above-mentioned duty of explanation to patients (or guardians of patients) in a wide range of ages smoothly while ensuring easy understanding. This can lead to the satisfaction of patients, etc. with the medical care provided by physicians.

As a specific example of the above-mentioned effect, the body fluid analysis device of the present invention displays "judgment of bacterial infection with 90% probability" when a blood sample indicates a white blood cell (WBC) count of 10000 cells/$\mu$l and a CRP value of 3 mg/dl, according to a given calculation formula (corresponding to the below-mentioned "formula ii"). Thus, it is extremely patient-friendly because one's own medical condition can be understood with ease.

According to the present invention, moreover, test values of plural items such as white blood cell count/lymphocyte count, platelet count, CRP value, and the like are used as explanatory variables for a multivariate analysis, and are associated with each other to determine severity of the condition of a patient with a viral infection including COVID-19 (objective variable). This increases the possibility of obtaining more correct differentiation results than when associating each test value independently for differentiation, and affords an objective judgment on the necessity of therapeutic intervention.

[Brief Description of Drawings]

[Fig. 1]

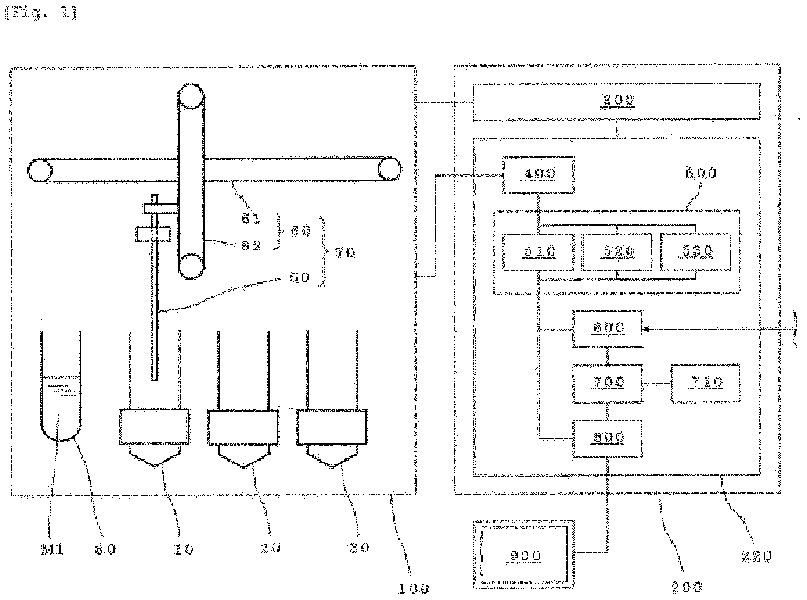

Fig. 1 is a block diagram showing one embodiment of a preferred configuration of the blood analysis device of the present invention.
[Fig. 2]

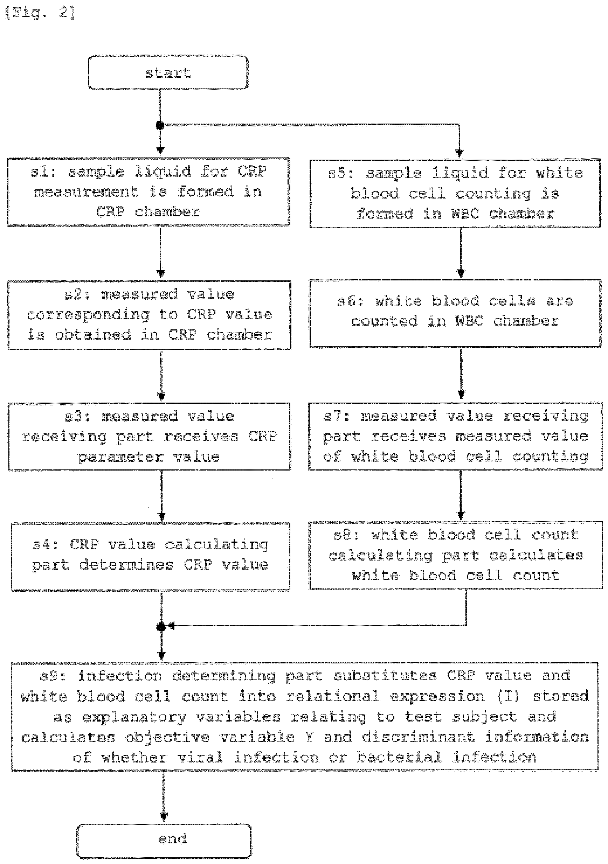

Fig. 2 is a flowchart showing an outline of the operation of the blood analysis device shown in the embodiment of Fig. 1. The flowchart also explains the constitutions of the method of the present invention and the computer program of the present invention.
[Fig. 3]

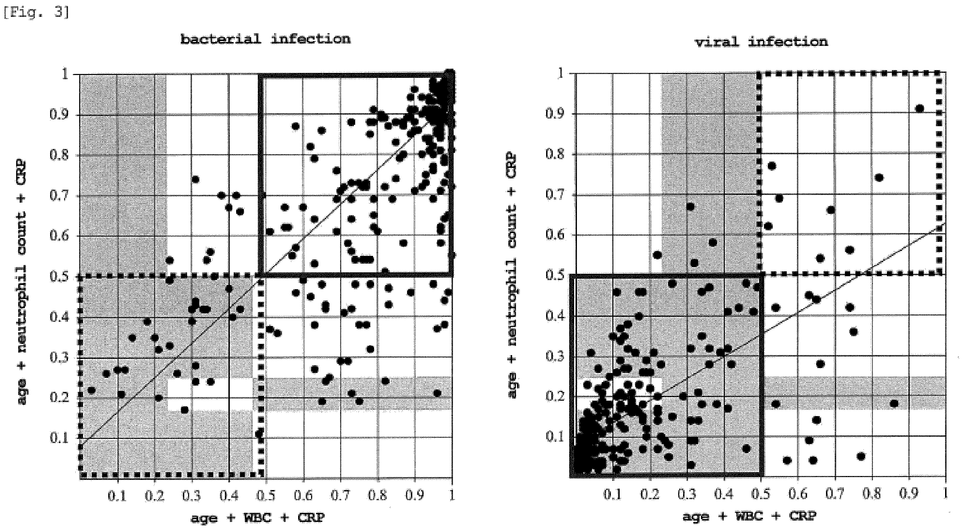

Fig. 3 shows a comparison of the judgment results of bacterial infection or viral infection of the test subjects, using relational expressions of two types of multiple logistic regression created using two parameter combinations (age + WBC + CRP and age + neutrophil + CRP) as explanatory variables. The left graph shows cases with a confirmed diagnosis of bacterial infection by the PCR test, and the right graph shows cases with a confirmed diagnosis of viral infection by the PCR test. The numerical values on each axis indicate not less than 0 and less than 0.5: viral infection, above 0.5 and not more than 1: bacterial infection.

[Description of Embodiments]

(Exemplary configuration of the body fluid analysis device of the present invention)

The body fluid analysis device of the present invention (hereinafter also to be referred to as the device) may be an analysis device (data processing device) provided only with a parameter receiving part and an infection determining part. As shown in the embodiment of Fig. 1, it is preferable to further have a configuration (mechanism and data processing function) for receiving a container containing a body fluid sample and performing a complete blood count analysis and a CRP value analysis. The body fluid sample that can be used in the device is not particularly limited as long as it is a body fluid derived from a test subject, and examples thereof include blood, ascites, pleural effusion, cerebrospinal fluid, and the like. Blood is preferred. In the present specification, the body fluid analysis device of the present invention, the method for testing a body fluid sample, and the computer program therefor are described in the following by taking blood, which is a representative example of body fluid, as an example. Therefore, the body fluid analysis device is sometimes referred to as a blood analysis device, the body fluid measuring mechanism is sometimes limitatively referred to as a blood measuring mechanism, the body fluid test item is referred to as a blood test item, and the body fluid sample is referred to as a blood sample, However, the present invention is not limited to blood but clearly includes embodiments using other body fluids as samples.
In the following description, the blood analysis device is described in detail with reference to the configuration thereof shown in Fig. 1. A description of each component included in the device is also a description of each component included in the method of the present invention and the computer program of the present invention.
As shown in Fig. 1, the device has at least a parameter receiving part 600 and an infection determining part 700.
The parameter receiving part 600 is a part that receives plural kinds of parameters relating to a test subject, that are used for the determination of (a) whether a test subject is suffering from a viral infection or a bacterial infection, or (b) whether a test subject suffering from a viral infection is in a severe condition. The plural kinds of parameters essentially include CRP value of a body fluid sample collected from the test subject, and also test value(s) of the body fluid sample that is(are) one or more body fluid test items selected from body fluid test items other than the CRP value. In the present invention, a multivariate analysis is used for determination of having a disease or severity thereof, and there are plural (that is, at least two) variables (parameters) to be multivariately analyzed. Representative examples of the plural kinds of parameters include, but are not limited to, CRP value and white blood cell count. The details of the plural kinds of parameters are described later.
On the other hand, the infection determining part 700 is a part configured to calculate determination information of (a) whether a test subject is suffering from a viral infection or a bacterial infection or (b) whether a test subject suffering from a viral infection is in a severe condition, based on the aforementioned plural kinds of parameters and using the below-mentioned relational expression (I) determined by a multivariate analysis and stored in advance. With the above configuration, the device can give more credible determination information as to whether the patient is (a) suffering from a viral infection or a bacterial infection, or (b) suffering from a severe viral infection, based on the blood sample of the patient.

(Plural kinds of parameters)

The plural kinds of parameters used in the present invention are plural explanatory variables necessary for a multivariate analysis. As mentioned above, the essential parameter is the CRP value, and other parameters include test value(s)of one or more body fluid test items selected from body fluid test items other than the CRP value. The test values of the one or more blood test items include white blood cell count, lymphocyte count, granulocyte count, neutrophil count, (neutrophil/lymphocyte ratio), (granulocyte count/lymphocyte ratio), platelet count, and the like. Among these test values, the white blood cell count can be mentioned as a test value that can provide more accurate differentiation when combined with the CRP value. When differentiating (a) whether a test subject is suffering from a viral infection or a bacterial infection, preferable combinations include CRP value and neutrophil count, CRP value and granulocyte count, CRP value and white blood cell count and neutrophil count, CRP value and white blood cell count and granulocyte count, CRP value and white blood cell count and neutrophil count and granulocyte count. On the other hand, when differentiating (b) whether a test subject suffering from a viral infection is in a severe condition, preferable combinations also include CRP value and white blood cell count and platelet count, CRP value and lymphocyte count and platelet count, CRP value and white blood cell count and lymphocyte count and platelet count.

In addition to the above-mentioned combination of CRP value and test value, further parameters can be used as explanatory variables in order to further increase the credibility of the differentiation results. Such additional parameters include one or more selected from age, gender, elapsed time since onset, and the like.

The age of the patient to whom the determination according to the present invention should be applied is not particularly limited. When differentiating whether a test subject is suffering from a viral infection or a bacterial infection, it is less than 6 years old in a preferred embodiment. In this case, a mode in which a message or warning such as (cannot be calculated because the age is not less than 6 years) is displayed, or a mode in which an error is displayed may be adopted when a numerical value greater than a predetermined age threshold value (e.g., 5 (years old)) is entered as a numerical value for age as a parameter. The same applies to other additional parameters. For each parameter, an appropriate range is determined in advance, and if a parameter outside that range is entered, the above message, warning, or error, and the like may be displayed. These indications may be by words, lamps, and the like.

As the format of the parameters processed in the device, they may be converted into numerical values with predetermined units so that humans (users such as physicians and patients) can generally refer to, such as the results of medical examinations and blood test results. They may also be signal values or data before conversion that can be used for arithmetic processing inside the device.

(Parameter receiving part 600)

The parameter receiving part receives the above-mentioned plural kinds of parameters and delivers them to the infection determining part 700. In the embodiment of Fig. 1, as a preferred embodiment, the device preferably has a blood measuring mechanism 100 and a measured value processing part 500, and is configured to be able to calculate at least the white blood cell count and CRP value from a blood sample collected from a test subject. In the embodiment of Fig. 1, the parameter receiving part 600 is configured to receive white blood cell count and CRP value from the measured value processing part 500. The blood measuring mechanism 100 and the measured value processing part 500 are configured to obtain additional blood test values such as neutrophil count, lymphocyte count, platelet count, and the like, and the parameter receiving part 600 may be configured to receive the additional blood test values. The configuration example shown in Fig. 1 is merely a preferred example, and the plural kinds of parameters received by the parameter receiving part 600 may be the CRP value and the above-mentioned test values. Furthermore, one or more parameters selected from age, gender, elapsed time since onset, and the like may be added.

The test values of a blood sample collected from a test subject may be values analyzed by an external blood analyzer. In that case, the parameter receiving part 600 may receive the test values from the Internet, LAN, and various data communication interfaces (not shown). In the embodiment of Fig. 1, an arrow line connected to the parameter receiving part 600 represents a signal line for receiving parameters from the outside.

It may be configured that a label affixed to a sample container containing a blood sample indicates parameters other than the test values, such as the age, gender and the like of test subject using a barcode or the like (not shown) so that the blood measuring mechanism 100 can read the parameters indicated on the label, and the parameter receiving part 600 receives the read parameters. These parameters may be received from the outside via the above-mentioned communication port. In addition, the device may be provided with various input devices (not shown) such as keyboard, touch panel, and the like. The parameter receiving part 600 may receive additional parameters useful for differentiation, such as the age and the gender of the test subject, elapsed time since onset, and the like from the input device.

(Infection determining part 700)

The infection determining part 700 is a part configured to calculate determination information for differentiation as to whether a test subject is (a) suffering from a viral infection or a bacterial infection, or (b) suffering from a severe viral infection, by using the following relational expression (I) stored in advance and based on the above-mentioned plural kinds of parameters (e.g., CRP value and other test values (information and values obtained from an automatic blood cell counting device such as white blood cell count (WBC), neutrophil count (NEU#), lymphocyte count (LYM#), platelet count (PLT), total nucleated cell count (TNC), red blood cell count (RBC), hemoglobin concentration (HGB), hematocrit value (HCT), mean red blood cell volume (MCV), mean corpuscular hemoglobin (MCH), mean corpuscular hemoglobin concentration (MCHC), red cell distribution width (RDW-CV, RDW-SD), platelet distribution width (PDW), plateletcrit value (PCT), mean platelet volume (MPV), microcytic red blood cell ratio (MIC%), macrocytic red blood cell ratio (MAC%), lymphocyte ratio (LYM%), monocyte count (MON#), monocyte ratio (MON$), neutrophil ratio (NEU%), eosinophils number (EOS#), eosinophils ratio (EOS%), basophil count (BAS#), basophil ratio (BAS%), atypical lymphocyte count (ALY#), atypical lymphocyte ratio (ALY%), large immature cell count (LIC#), large immature cell ratio (LIC%), immature lymphocyte count (IML#), immature lymphocyte ratio (IML%), immature monocyte count (IMM#), immature monocyte ratio (IMM$), immature granulocyte count (IMG#), immature granulocyte ratio (IMG%), nucleated red blood cell count (NRBC#), nucleated red blood cell ratio (NRBC%), body fluid red blood cell count (BFRBC), body fluid white blood cell count (BFWBC), body fluid monocyte count (BFMN#), body fluid monocyte ratio (BFMN%), body fluid polymorphonuclear cell count (BFPN#), body fluid polymorphonuclear cell (BFPN%), platelet count by optical measurement (PLTOPT), reticulocyte count (RET#), reticulocyte ratio (RET$),low fluorescence reticulocyte ratio (RETL$), medium fluorescence reticulocyte ratio (RETM%), high fluorescence reticulocyte ratio (RETH%), corrected reticulocyte count (CRC), mean reticulocyte volume (MRV), mean reticulocyte fluorescence index (MFI$), immature reticulocyte fraction (IRF), reticulocyte intracellular hemoglobin concentration (RHCc), mean corpuscular fluorescence level (PIC), and the like), age, and the like). The detail of the infection determining part is described below.

In the present invention, the "determination of whether a test subject is suffering from a viral infection or a bacterial infection" means, in the preferred embodiment of the present invention, (determination of whether a test subject is suffering from one of viral infection and bacterial infection). Similarly, in the present invention, the "determination information as to whether each of the plurality of data providers was suffering from a viral infection or a bacterial infection" is information when, in the preferred embodiment of the present invention, each data provider was suffering from one of viral infection and bacterial infection. Therefore, it is preferable to exclude the determination or the information of being suffering from both.

However, in other embodiment of the present invention, a case may be added to the aforementioned alternative determination in which plural kinds of parameters are analyzed in more detail, and depending on the results, it is determined that (the test subject is suffering from both a viral infection and a bacterial infection). In this case, the determination is (the test subject is highly possibility suffering from a bacterial infection), (the test subject is highly possibility suffering from a viral infection), (the test subject is highly possibility suffering from both a bacterial infection and a viral infection), or the like. In this case, the "determination information as to whether each of the plurality of data providers was suffering from a viral infection or a bacterial infection" may include the information that each data provider was suffering from both viral infection and bacterial infection.

In the present invention, the "determination of whether a patient with a viral infection is severely ill(whether a test subject is suffering from a severe viral infection)" means determination of, in a preferred embodiment of the present invention, whether a test subject who is a patient with a viral infection is suffering from a severe viral infection or suffering from a non-severe (mild or moderate) viral infection. Similarly, in the present invention, the "determination information as to whether each of the plurality of data providers was suffering from a severe viral infection or the same but non-severe viral infection" is information when, in the preferred embodiment of the present invention, each data provider was suffering from one of a severe viral infection and a non-severe (mild or moderate) viral infection.

Being "severe" or "non-severe" is classified according to the criteria for determining clinical severity, depending on the targeted viral infection. Viral infections whose severity can be differentiated by the present invention are not particularly limited. In a preferred one embodiment, it is SARS-CoV-2 infection (COVID-19). In the case of COVID-19, the "severe condition" means that the patient is in a state of respiratory failure to the extent that artificial breathing management and intensive care with ECMO are required. The degree of respiratory failure is generally assessed by oxygen saturation (SPO2). In the below-mentioned Example, SPO2 of less than 94 is defined as severe, and 94 or more is defined as mild (not severe).

(Relational expression (I))

When differentiating whether a test subject is suffering from a viral infection or a bacterial infection, the relational expression (I) can be determined in advance by a multivariate analysis by the procedures of (a1) and (a2).

(a1) The same plural kinds of parameters as the above-mentioned plural kinds of parameters are obtained from each of the plurality of data providers known to be suffering from a viral infection and the plurality of data providers known to be suffering from a bacterial infection. For example, when a CRP value and a white blood cell count were obtained as the plural kinds of parameters from the aforementioned data providers in advance and relational expression (I) was constructed and stored, the determination information relating to the differentiation is calculated from a CRP value and a white blood cell count of a blood sample of a patient also in the infection determining part 700.

(a2) The aforementioned plural kinds of parameters are referred to as "explanatory variables". On the other hand, the determination information relating to the differentiation of whether the aforementioned data providers were each suffering from a viral infection or a bacterial infection is referred to as "objective variable". Then, by a multivariate analysis, a relational expression (e.g., multiple regression equation, multiple discriminant equation, and the like) showing the relationship between the "explanatory variable" and the "objective variable" is determined.

When additional parameters other than the CRP value and the above-mentioned test value are added as explanatory variables, as described in the above-mentioned (a1), (a2), the additional parameters are obtained from each of the plurality of data providers known to be suffering from a viral infection and the plurality of data providers known to be suffering from a bacterial infection and used as explanatory variables together with the CRP value and test values such as white blood cell count and the like.

When differentiating whether a patient with a viral infection is in a severe condition, the relational expression (I) can be determined in advance by a multivariate analysis by the procedures of (b1) and (b2).

(b1) The same plural kinds of parameters as the above-mentioned plural kinds of parameters are obtained from each of the plurality of data providers known to be suffering from a severe viral infection and the plurality of data providers known to be suffering from the same but non-severe viral infection. For example, when a CRP value, a white blood cell count, and a platelet count were obtained as the plural kinds of parameters from the aforementioned data providers in advance and relational expression (I) was constructed and stored, the determination information relating to the differentiation is calculated from a CRP value, a white blood cell count, and a platelet count of a blood sample of a patient also in the infection determining part 700.

(a2) The aforementioned plural kinds of parameters are referred to as "explanatory variables". On the other hand, the determination information relating to the differentiation of whether the aforementioned data providers were each suffering from a severe viral infection or the same but non-severe viral infection is referred to as "objective variable". Then, by a multivariate analysis, a relational expression (e.g., multiple regression equation, multiple discriminant equation, and the like) showing the relationship between the "explanatory variables" and the "objective variable" is determined.

When additional parameters other than the CRP value and the above-mentioned test values are added as explanatory variables, as described in the above-mentioned (b1), (b2), the additional parameters are obtained from each of the plurality of data providers known to be suffering from a severe viral infection and the plurality of data providers known to be suffering from the same but non-severe viral infection, and used as explanatory variables together with the CRP value and test values such as white blood cell count and the like.

The relational expression (I) is stored in a storage device within the device or an external communicable storage device such that the infection determining part 700 can refer to. In the embodiment of Fig. 1, the relational expression (I) is stored in a relational expression holding part 710 provided in one area of the storage device within the device.

(Multivariate analysis for determining relational expression (I))

A specific analysis method itself for a multivariate analysis is not particularly limited. For example, one or more analysis methods selected from a discriminant analysis, a logistic regression analysis, a multiple regression analysis, a Cox hazard regression analysis (also called Cox proportional hazards analysis and the like), a principal component analysis, a factor analysis, a variance analysis, and a cluster analysis are preferred. In the present invention, one of these analysis methods is preferably performed, and two or more analysis methods may be selected and conducted to confirm determination and increase the accuracy of the determination.

(Discriminant analysis)

In the discriminant analysis of whether a test subject is suffering from a viral infection or a bacterial infection, for example, among the blood test values of blood samples collected from each of a plurality of data providers proven to have viral infection (Dw), and a plurality of data providers proven to have bacterial infection (Db), white blood cell count X1 and CRP value X2 are explanatory variables, and the binary value of viral infection or bacterial infection is an objective variable (Y1). For example, viral infection is 0 and bacterial infection is 1. Then, the regression coefficients ($a_1$, $a_2$, $a_0$) are calculated using the following formula (i) as the relational expression (I) .

$$Y1 = a_1 X1 + a_2 X2 + ... + a_0 \qquad \text{formula (i)}$$

$a_1 X1$ is a product of $a_1$ and X1. $a_0$ is also referred to as a constant term.

When the above-mentioned further parameters X3, X4, ... are added as explanatory variables, $(a_3 \times X_3 + a_4 \times X4 + ...)$ is added on the right side of the above-mentioned formula (i).

The regression coefficient of the relational expression is determined so as to maximize the correlation ratio of, for example, the actual differential value of the data provider (viral infection or bacterial infection) and the value obtained by applying each parameter (explanatory variable) of the data provider to the relational expression.

In the discrimination of a blood sample collected from a test subject, for example, the test values (white blood cell count, CRP value) are entered in the above-mentioned formula (i), and if Y1<0, determination information that the test subject highly likely has a viral infection is output, and if Y1>0, determination information that the test subject highly likely has a bacterial infection is output.

In addition, if the calculation results using the relational expression (I) are obtained as the probability (%) of bacterial infection and the probability (%) of viral infection, one with a calculation result (determination result) exceeding 50% may be determined to have infection. For example, it may be configured that when the probability of bacterial infection is calculated to be 55% and the probability of viral infection is calculated to be 450, then determination information that the test subject has high possibility of bacterial infection is output.

In the aforementioned example, the discriminant information for dividing into two groups of viral infection and bacterial infection was calculated. The discriminant information may be an information for dividing into three or more groups, for example, 4 groups (1. suffering from a viral infection alone, 2. suffering from a bacterial infection alone, 3. suffering from both a viral infection and a bacterial infection, 4. not suffering from viral infection or bacterial infection) and the like. In this case, the parameters may be collected from data providers proven to belong to the respective groups, and the above-mentioned relational expression (I) may be calculated. Where necessary, multiple relational expressions may be used.

(Logistic regression analysis)

In a logistic regression analysis (also referred to as multiple logistic regression analysis), a relational expression previously calculated based on the parameters from the above-mentioned data provider is used to calculate the probability whether the test subject has a viral infection or a bacterial infection. The parameters from the data provider and the like used for a logistic regression analysis are the same as those in the above-mentioned discriminant analysis. The relational expression (I) of the logistic regression analysis is represented by the following expression (ii).

$$Y2 = 1/\{1 + \exp[-(a_1 X1 + a_2 X2 + ... + a_0)]\} \qquad \text{(formula ii)}$$

The respective number of past data providers (Dw, Db) in the multivariate analysis exemplified above is not particularly limited. For example, it is preferably a number capable of performing k-fold cross-validation (e.g., 2-fold, 3-fold, 5-fold, etc.). In the Example of the present invention, 260 cases of data provider Dw, 229 cases of data provider Db, less than one year to 16 years of age, 258 male cases, 231 female cases were used, and these are sufficient numbers to obtain relational expressions with high credibility.

Differentiation of whether a patient with a viral infection is severely ill can also be performed in the same manner as described above. For example, among the blood test values of blood samples collected from each of a plurality of data providers proven to have severe viral infection (Dw), and a plurality of data providers proven to have the same but non-severe viral infection (Db), white blood cell count X1, CRP value X2, and platelet count X3 are explanatory variables, and severe or non-severe are objective variables (Y1, Y2). Then, the regression coefficients $(a_1, a_2, a_3, a_0)$ are calculated using the above formula (i) or (ii) as the relational expression (I). The regression coefficient of the relational expression is determined so as to maximize the correlation ratio of, for example, the actual differential value of the data provider (severe or non-severe) and the value obtained by applying each parameter (explanatory variable) of the data provider to the relational expression.

In the discrimination of a blood sample collected from a test subject, for example, the test values (white blood cell count, platelet count, CRP value) are entered in the above-mentioned formula (i) or (ii), Y1 or Y2 value is determined, and determination information that the test subject is highly possibly in a severe condition or not in a severe condition can be obtained.

Similar to the determination of the kind of infection, the discriminant information of the severity of viral infection may be information for dividing into three or more groups, for example, 4 groups (1. severe, 2. moderate, 3. mild, 4. asymptomatic) and the like. In this case, the parameters may be collected from data providers proven to belong to the respective groups, and the above-mentioned relational expression (I) may be calculated. Where necessary, multiple relational expressions may be used.

In the above-mentioned multivariate analysis, as explanatory variables, blood test items other than CRP value, and additional parameters such as age, gender, elapsed time since onset, and the like are replaced with other parameters and two kinds of relational expressions (I) are created. Using them, differentiation of test subjects is performed, and the results are compared, whereby erroneous determination can be reduced. For example, in the differentiation of whether the test subject has a viral infection or a bacterial infection, relational expression (I) for a logistic regression analysis is created for each of (1) when CRP value and white blood cell count and age are explanatory variables, and (2) when CRP value and neutrophil count and age are explanatory variables, enter the test values and the like of the test subject in each relational expression (I), and the differentiation results are compared (Fig. 3). As a result, the determination does not match between (1) and (2) in both bacterial infection and viral infection (upper left part and lower right part when each graph in Fig. 3 is divided into 4 parts), and there are cases where the determination of (1) is correct (the lower right part of the left graph and the upper left part of the right graph) and the case where the determination of (2) is correct (the upper left part of the left graph and the lower right part of the right graph). When the determination is different between (1) and (2), the frequency of erroneous determination can be reduced by withholding the determination and relying on the confirmed diagnosis by PCR test or the like.

(Blood measuring mechanism)

In the embodiment shown in Fig. 1, the device further has the blood measuring mechanism 100. The blood measuring mechanism 100 is configured to receive a sample container 80 containing a blood sample M1 at a predetermined position, measure the blood sample M1 in the sample container X1, and deliver the obtained measurement signals to the measured value processing part 500. Since CRP value is essential for differentiation and white blood cell count is preferred as other blood test item, the blood measuring mechanism 100 is preferably provided with a white blood cell measuring chamber (hereinafter also to be referred to as WBC chamber) 30 for measuring the white blood cell count of the blood sample M1 and a CRP measuring chamber (hereinafter also to be referred to as CRP chamber) 10 for measuring the CRP value of the blood sample M1. The embodiment of Fig. 1 also includes a red blood cell measuring chamber (also to be referred to as RBC chamber) 20, like the blood analysis device of the above-mentioned Patent Literature 1. As described below, the RBC chamber 20 is a chamber configured to count red blood cells and platelets. For differentiation of the severity of viral infection of patients, it is preferable to use the platelet count as an explanatory variable in addition to the CRP value and the white blood cell count. Therefore, the blood measuring mechanism 100 is desirably provided with an RBC chamber.

Counting in the following explanation includes not only simply counting the number of blood cells such as white blood cell and the like, but also measuring the volume of each blood cell. This makes it possible to analyze blood cells of what volume are present in what number (that is, volume frequency distribution of blood cells). The "frequency" in the volume frequency distribution is the number of particles per each volume (or channel).

It is preferable to divide the overall width from the minimum value (may be zero) to the maximum value of the measured volume value showing the volume of blood cells into, for example, 256 steps (0 to 255), 1024 steps (0 to 1023) and the like rather than the specific numerical values. This facilitates handling of the data when the below-mentioned analysis part processes the data to determine the frequency distribution. Each section divided as mentioned above is also called a channel.

(WBC chamber 30)

In the WBC chamber 30, the blood sample M1 is subjected to a hemolysis treatment, a diluent solution is added to form a sample liquid, white blood cells in the sample liquid are counted, and the signal at the time of counting is transmitted. The signals may be processed by an analysis part 220. The WBC chamber 30 may be configured to perform at least white blood cell counting by, for example, an impedance method. In a preferred embodiment as a blood analysis device, additional reagents are added to the sample liquid for treating white blood cells so as to permit classification into lymphocytes, monocytes, and granulocytes. By analyzing the signal data indicating the volume of each particle obtained from the WBC chamber 30, the frequency distribution of lymphocytes, monocytes, and granulocytes can be obtained. For example, the aforementioned diluent solution also exhibits the action of protecting the cellular membrane of white blood cells. Hemolytic agents destroy red blood cells and platelets and exert a contractile action on three types of white blood cells (lymphocytes, monocytes, granulocytes). In lymphocytes, the hemolytic agent causes dehydration from within the cytoplasm, leading to the contraction (naked nucleation) of nuclear membrane. Monocytes and granulocytes show different degrees of naked nucleation, and granulocytes are smaller than monocytes. Utilizing these phenomena, therefore, lymphocytes, monocytes, and granulocytes can be distinguished (three classifications) based on particle size. In addition, the WBC chamber 30 may also be configured to measure hemoglobin concentration (Hgb).

(RBC chamber 20)

The RBC chamber 20 is a chamber configured to count red blood cells and platelets based on an impedance method or the like. By analyzing the counting results, the volume frequency distribution of red blood cells and platelets can also be obtained. The hematocrit value (Hct) is calculated by dividing the total volume of red blood cells in the sample obtained by an impedance method or the like by the total volume of blood.

(Chamber for measuring neutrophil count)

As a chamber for measuring neutrophil counts, a measuring chamber configured to treat white blood cells with a reagent into countable particles of lymphocytes, monocytes, neutrophils, and eosinophils, and count the particles can be mentioned. As such measuring chamber, LMNE cell (LMNE chamber) described in the above-mentioned Patent Literature 2 can be mentioned. In the LMNE chamber, particles are counted using a reagent and a particle counting mechanism (particularly, mechanism for light-focused flow impedance method). By analyzing the counting results of the particles, the frequency distribution of lymphocytes, monocytes, neutrophils, and eosinophils can be obtained and neutrophil count can also be obtained. As mentioned above, a relational expression showing the relationship to the objective variables (viral infection and bacterial infection) is determined using the CRP value and the white blood cell count as explanatory variables. In addition, a relational expression showing the relationship to the objective variables (viral infection and bacterial infection) is similarly determined using the CRP value and the neutrophil count as explanatory variables, and the differentiation results of the test subject are compared, whereby erroneous determination can be reduced. Thus, the blood measuring mechanism 100 is desirably further provided with a chamber for measuring the neutrophil count.

(Chamber for measuring basophil)

In addition to the aforementioned LMNE chamber, a measuring chamber (BASO chamber) for counting basophils may be provided. This enables five classifications of white blood cells and more detailed blood analysis. In the BASO chamber, particles components other than basophil are hemolysed and contracted with a hemolytic agent, and basophils are counted by a particle counting mechanism.

(CRP chamber 10)

CRP chamber is configured to measure a CRP parameter value which is an indicated value corresponding to the CRP value of the blood sample M1, and transmit the measured value. Examples of the method for obtaining the CRP parameter value in a measurement chamber include CRP latex immunity nephrometry RATE method and the like. As the configuration of the CRP chamber, for example, a photoirradiation part and a photodetection part are provided on the lower wall surface of the chamber, and the intensity of the detected transmitted light or scattered light is obtained. In an example configuration for performing the CRP latex immunoturbidimetric RATE method, a blood sample is mixed with anti-human C-reactive protein (CRP) antibody-sensitized latex in the presence of a buffer solution, a rate of coagulation caused by an antigen-antibody reaction of the CRP in the sample and the aforementioned latex particles is measured, and the CRP concentration is determined using the rate of coagulation as a variable and a polynomial calibration curve prepared in advance based on the standard serum. When the whole blood is used as a blood sample, for example, the whole blood is previously hemolyzed with a hemolytic agent and used as a sample, and the hematocrit value calculated above is converted into the plasma CRP concentration of the test subject and displayed. A series of CRP measurements are performed in a CRP value calculating part 510 of the below-mentioned analysis part 220.
Regarding the CRP value, in test subjects (e.g., patients) with a hematocrit value, which is the percentage of red blood cells contained in a given amount of blood, exceeding a certain value (e.g., 55%), sodium citrate concentration increases since the amount of plasma derived from the test subject is small. Therefore, when obtaining this value, hematocrit correction may be performed by adjusting the amount of sodium citrate solution by a given method in consideration of the CLSI guidelines. Specifically, for example, the correction may be made using a method including the following steps (i) to (iii):

(i) a step of aggregating latex particles by hemolyzing the whole blood with a hemolytic agent, and then reacting same with an anti-human C-reactive protein (CRP) antibody-sensitized latex reagent in the presence of a buffer solution to cause an antigen-antibody reaction of CRP in the sample and the latex particles in the reagent,
(ii) a step of measuring the turbidity change rate due to the aggregation reaction with red light and determining the concentration of CRP in the hemolyzed sample by using a polynomial calibration curve prepared in advance based on the standard serum, and
(iii) a step of converting the hematocrit value measured at the same time into the plasma CRP concentration of the test subject and displaying same.

Examples of the particle counting mechanism in each of the above-mentioned measuring chamber include

a mechanism of performing an impedance method (electrical resistance method) (mechanism of measuring volume of particles by using a change in the electric property (impedance) in aperture when the particles pass through the aperture),

a mechanism of performing flow cytometry (irradiating predetermined irradiation light to particles in a sample liquid advancing through a flow path in a flow cell, obtaining optical properties such as light scattering, light absorbance and the like, and measuring the volume of the particles from the optical properties),

a light-focused flow impedance method (mechanism for performing an impedance method and flow cytometry in one flow path so that each particle can be measured by an impedance method and flow cytometry),

and the like.

For the techniques for counting and analysis of white blood cell count and red blood cell count, the techniques for further classification of white blood cells, and the technique itself for measuring the CRP value, conventionally-known techniques such as the above-mentioned Patent Literatures 1 and 2 and the like can be referred to.

(Dispensing mechanism and the like)

In the embodiment of Fig. 1, the blood measuring mechanism 100 has a dispensing mechanism 70 in addition to the CRP chamber 10, the RBC chamber 20, and the WBC chamber 30. The dispensing mechanism 70 has a sampling nozzle 50 and a transfer mechanism 60. The transfer mechanism 60 is configured to contain a horizontal transfer mechanism 61 and a vertical transfer mechanism 62 so that the sampling nozzle 50 can be moved in the horizontal direction and the vertical direction. The sampling nozzle 50 is an elongated tube under the control of a mechanism control part 300 contained in a control part 200 and moves to the sample container 80, various reagent containers (not shown), the CRP chamber 10, the RBC chamber 20, and the WBC chamber 30 to suck and discharge a blood sample, a reagent liquid, a diluent solution, a sample liquid produced in a chamber, and the like.

In addition to these, the blood measuring mechanism 100 is appropriately provided with a dilution mechanism, a washing mechanism, a pump for sucking/discharging, a syringe, an electromagnetic valve, and the like (not shown). For the blood measuring mechanism, conventionally-known particle analysis devices, blood analysis devices, blood cell count devices, and the like can be referred to.

(Control part 200)

In the embodiment of Fig. 1, the control part 200 has the mechanism control part 300 and the analysis part 220 which is the main part for calculation. The mechanism control part 300 controls and operates the blood measuring mechanism 100. The control part 200 may be appropriately connected to an external driving circuit and the like for controlling the blood measuring mechanism. The analysis part 220 receives identification information of a test subject read from the sample container 80 by the blood measuring mechanism 100, receives measurement result signals from the blood measuring mechanism 100, calculates blood test value of the sample, calculates determination information for differentiation, and outputs these blood test values and the determination information. In the embodiment of Fig. 1, the analysis part 220 is a part that receives, processes, and outputs data for calculation of determination information for the analysis and differentiation of blood, and includes a measured value receiving part 400, the measured value processing part 500, the parameter receiving part 600, the infection determining part 700, a calculation result output part 800, and the like.

The control part 200 may be constructed with a logic circuit and the like, but suitably constructed with a computer and a computer program. In the computer, a computer program configured to control action of each part of the blood measuring mechanism 100 and perform calculation of blood test values and determination information for differentiation is executed.

(Measured value receiving part 400)

In the embodiment of Fig. 1, the measured value receiving part 400 receives measured values transmitted from each measurement part (10, 20, 30) in the blood measuring mechanism 100 as arithmetically processable data. The measured value receiving part 400 may be configured to contain an interface as a hardware, and a software (computer program) that accepts the received measured value signals as a data set of the measured values. The measured value receiving part 400 may store the received respective measured values in a storage device (not shown).

(Measured value processing part 500)

In the embodiment of Fig. 1, the measured value processing part 500 has the CRP value calculating part 510, a red blood cell count calculating part 520, and a white blood cell count calculating part 530. The red blood cell count calculating part 520 receives the measured values in the RBC chamber (data as measured) from the measured value receiving part 400, and calculates red blood cell count, platelet count, and volume frequency distribution thereof in the blood sample M1.

Similarly, a white blood cell count calculating part 530 receives the measured values in the WBC chamber from the measured value receiving part 400, and calculates white blood cell count and volume frequency distribution in the blood sample M1. When the white blood cells are treated so as to permit classification into lymphocytes, monocytes, and granulocytes, the white blood cell count calculating part 530 receives the measured values in the WBC chamber from the measured value receiving part 400, and further calculates lymphocyte count, monocyte count, granulocyte count, and volume frequency distribution thereof in the blood sample M1. The CRP value calculating part 510 receives the measured values (signal values sent from light receiving sensor) in the CRP chamber from the measured value receiving part 400, and determines the CRP value of the blood sample M1. When the blood measuring mechanism is further provided with, for example, a chamber for measuring neutrophils and the like, the measured value processing part 500 may further has a neutrophil count calculating part and the like. The neutrophil count calculating part receives the measured values in the chamber for measuring neutrophils from the measured value receiving part 400, and calculates neutrophil count volume frequency distribution in the blood sample M1.

(Calculation result output part 800)

The calculation result output part 800 is a part that outputs the calculated results (blood analysis results of conventional blood analysis device) in the measured value processing part 500, and determination information relating to the above-mentioned differentiation calculated in the infection determining part 700, to a display device 900, peripheral devices such as printer and the like, communication line, external computer, and the like.

[Main operation flow of the device]

Fig. 2 is a flowchart illustrating an outline of the operation of the device illustrated in Fig. 1. In the example of the flowchart, while analysis steps s1 to s4 of the CRP value and analysis steps s5 to s8 of the leukocyte count are shown in parallel, the operations of these two series may be serial. The number assigned to each step is for identification. In the embodiment of Fig. 1, an RBC chamber is added, blood samples are distributed to each chamber in a predetermined order to form sample liquids there (also including the transfer of sample liquids between chambers; for example, a portion of blood sample diluted in WBC chamber is transferred to RBC chamber where it is further diluted), after which measurement proceeds independently in each chamber and the time required for the measurement is also independent for each chamber. The operation of each part is based on the command from the control part. In the following, the measurement of these parameters and the flow of calculation of determination information using same are explained by referring to an example using CRP value and white blood cell count as the plural kinds of parameters.

(CRP value measuring steps s1 to s4)

(Step s1)

The dispensing mechanism 70 operates, the sampling nozzle (hereinafter nozzle) 50 moves to measure the CRP value, supplies predetermined amounts of a reagent necessary for the measurement (reagent container not shown) and a blood sample M1 in the sample container X1 into the CRP chamber 10, where a sample liquid for measuring the CRP value is formed. The nozzle is washed as appropriate.

(Step s2)

A measured value (absorbance) corresponding to the CRP value is obtained in the CRP chamber, and the measured value is sent to the analysis part 220.

(Step s3)

The measured value receiving part 400 in the analysis part 220 receives the measured value of CRP from the CRP chamber receives.

(Step s4)

The CRP value calculating part 510 of the measured value processing part 500 receives measured values of CRP from the measured value receiving part 400 and determines CRP value. The CRP value calculating part 510 may have a reference table for converting the measured value of CRP to CRP value. The CRP value is sent to the parameter receiving part 600.

(White blood cell count measuring steps s5 to s8)

(Step s5)

The dispensing mechanism 70 operates, and the nozzle 50 supplies a predetermined amount of the blood sample M1 in the sample container 80 to the WBC chamber 30, and a predetermined amount of a diluent solution (water, saline, phosphate buffer diluent or the like) is supplied thereto to dilute the blood sample M1. While the flowchart of Fig. 2 does not specifically show the detailed action, a part of the blood sample diluted in the WBC chamber is transferred to the RBC chamber, further diluted therein, and erythrocytes and platelets are counted. For the dilution rates of these, conventionally-known counting techniques can be referred to. In the WBC chamber, a hemolytic agent that further dissolves erythrocytes (hemolysis) is added to the diluted blood sample to form a sample liquid in which erythrocytes are dissolved.

(Step s6)

White blood cells are counted in the WBC chamber 30, the volume of each white blood cell is measured, and the measured values (size signals corresponding to the volume) are sent to the analysis part 220. In the WBC chamber, a hemoglobin concentration is measured by an optical device for a colorimetric method (non-cyan method), and the measured values may be sent. In the WBC chamber 30, processing to further classify white blood cells into lymphocyte, monocyte, and granulocyte may be performed. In the RBC chamber 20, the particles are also counted, the volume of particles containing red blood cell and platelet is measured, and the measured values are sent to the analysis part 220.

(Step s7)

The measured value receiving part 400 receives the measured value relating to white blood cell count from the WBC chamber receives.

(Step s8)

The white blood cell count calculating part 530 of the measured value processing part 500 receives the measured value of white blood cells from the measured value receiving part 400, and calculates a white blood cell count per a blood unit volume. The white blood cell count is sent to the parameter receiving part 600. Similarly, the red blood cell count calculating part 520 receives the measured values of red blood cells and platelets from the measured value receiving part 400, and calculates a red blood cell count and a platelet count per a blood unit volume. The calculation results in the measured value processing part 500 may be output through the calculation result output part 800, or may be displayed as histogram or scattergram on the display device 900 and the like.

(Calculation step of determination information for differentiation)

(Step s9)

The infection determining unit 700 receives the white blood cell count and CRP value from the parameter receiving part 600, these white blood cell count and CRP value as explanatory variables relating to the test subject are substituted into the above-mentioned relational expression (I) stored in the relational expression holding part 710, and the objective variable Y is calculated. According to the calculation results, determination information as to whether the test subject is suffering from a viral infection or a bacterial infection (when the objective variable is severity of patient with a viral infection, whether the test subject is severely ill) is selected (when the objective variable Y is an established value, it may be used as it is as determination information).

By the above steps, the determination information is preferably calculated. The calculated determination information is transmitted to the calculation result output part 800 and output on a display device and the like.

[Indication of determination information]

The indication of the results of the determination information obtained by the infection determining part 700 may be a sentence, a numerical value, a symbol, or the like that can be understood as a reference for the above-mentioned discrimination by the operator of the blood analysis device or those who see the analysis results, such as "high possibility of viral infection (or bacterial infection)", "- % possibility of viral infection (or bacterial infection) ", or "high possibility of severe viral infection (or non-severe viral infection)", and "- % possibility of severe viral infection (or non-severe viral infection)".

[Method of the present invention]

The method of the present invention is a method for determining whether a blood sample derived from a test subject is obtained from (a) a test subject with a viral infection or a test subject with a bacterial infection, or (b) a test subject suffering from a severe viral infection or a test subject suffering from the same but non-severe viral infection. The blood sample derived from a test subject is, for example, a blood sample collected from a test subject. The method of the present invention can be said to be a method for assisting (physicians and the like) to differentiate whether a test subject who is a provider of a blood sample has (a) viral infection (suffering from a viral infection), or a bacterial infection (suffering from a bacterial infection), or has (b) a severe viral infection(suffering from a severe viral infection) or a non-severe viral infection (suffering from a non-severe viral infection).

The method has a first step of preparing the above-mentioned plural kinds of parameters. The method for obtaining the plural kinds of parameters is not particularly limited, and the above-mentioned blood analysis device of the present invention is preferably used. In addition, the method has a second step of determining whether the aforementioned blood sample is obtained from (a) a test subject with a viral infection or a test subject with a bacterial infection, or (b) a test subject suffering from a severe viral infection or a test subject suffering from the same but non-severe viral infection by using the aforementioned plural kinds of parameters and the above-mentioned relational expression (I). The determination method using the above-mentioned relational expression (I) is as described in detail above.

The multivariate analysis used in the method of the present invention may be, as described above, one or more analyses selected from a discriminant analysis, a logistic regression analysis, a multiple regression analysis, a Cox hazard regression analysis, a principal component analysis, a factor analysis, a variance analysis, and a cluster analysis.

A test value of a blood sample relating to one or more blood test items selected from blood test items other than the above-mentioned CRP value is as described above.

[Computer program of the present invention]

The computer program of the present invention (hereinafter also to be referred to as the program) may be provided after recording on a computer readable medium, or may be provided through a network from other computer or external storage device.

The program is a computer program that makes a computer function as the above-mentioned parameter receiving part (i.e., parameter receiving means), and the above-mentioned infection determining part (i.e., infection determining means). The parameter receiving part and the infection determining part are as described in detail in the above.

In addition, the program may further include a computer program that makes a computer function as each part of the above-mentioned blood analysis device of the present invention (white blood cell count calculating part, CRP value calculating part, neutrophil count calculating part, control part, and the like) .

[Example]

Example 1: Outline of clinical test

The clinical test was conducted according to a given protocol approved by the review committee of the National Hospital Organization Tokyo Medical Center (J Infect Chemother (2012) 18:832-840).

Example 2: Setting of relational expression (I) by multivariate analysis

Among the samples obtained by the clinical test, the samples derived from infants under 7 years old were first classified by the PCR test into viral infection, bacterial infection, mixed (both viral and bacterial) infection, and mycoplasma infection (viral infection 260 cases, bacterial infection 229 cases, mixed infection 208 cases, mycoplasma infection 86 cases (total 783 cases)).

Next, statistical analysis was performed on the samples mentioned above by using StatFlex (Artec Co., Ltd.).

Specifically, using StatFlex multivariate analysis (multiple logistics regression), viral infection, bacterial infection, mixed infection, and mycoplasma infection by PCR were selected as objective variables, age, WBC, CRP, gender, sick days, fever days, neutrophil, severity, and viral load were selected as explanatory variables, and it was evaluated and examined which combination would maximize the area under the ROC curve (AUC).

The results are shown in the following Table 1.

[Table 1]

| order | variable name | $\beta$ (regression coefficient) | SE ($\beta$) | z | P | odds ratio | 95% CI lower limit | 95% CI upper limit |
|---|---|---|---|---|---|---|---|---|
| 0 | | -8.175 | 0.6667 | | | | | |
| 1 | age (years old) | -0.023 | 0.05031 | -0.457 | 0.64753 | 0.977 | 0.886 | 1.079 |
| 2 | WBC (/$\mu$l) | 5.41E-04 | 4. 61E-05 | 11.721 | 0 | 1.001 | 1 | 1.001 |
| 3 | CRP (mg/dl) | 0.8445 | 0.08176 | 10.329 | 0 | 2.327 | 1.982 | 2.731 |

Using $\beta$ values in Table 1 as $a_0$ to as (regression coefficients) in the above-mentioned relational expression (I) (formula (ii)), the formula can be set as the formula (ii) of the present invention.

Example 3: Evaluation of relational expression (I) by K-fold cross-validation

The following validations 1 and 2 were performed in order to verify and confirm the validity of the setting of the formula (ii) of the present invention which was set by the aforementioned method.

(1) Validation 1

In the method of validation 1, using the raw data obtained by the above-mentioned clinical test (J Infect Chemother (2012) 18:832-840), verification and confirmation were performed by the following methods:

(i) divide into 5, create a calculation formula (i.e., corresponding to formula (ii) of the present invention) for one of the divisions, and evaluate all 5 divisions,
(ii) divide into 3, create a calculation formula (i.e., corresponding to formula (ii) of the present invention) for one of the divisions, and evaluate all 3 divisions (see Table 3 for the results), and
(iii) divide into 2, create a calculation formula (i.e., corresponding to formula (ii) of the present invention) for one of the divisions, and evaluate all 2 divisions (see Table 4 for the results).

(i) The 5-fold validation results are shown in the following Table 2.

[Table 2]

| | 5-fold | | | |
|---|---|---|---|---|
| average | 0.900 | 0.905 | 0.956 | 0.966 |
| SD | 0.011 | 0.040 | 0.008 | 0.016 |
| CV | 1.2% | 4.4% | 0.8% | 1.7% |

| | concordance rate | | AUC | |
|---|---|---|---|---|
| | for learning | for verification | for learning | for verification |
| 1 | 0.8914 | 0.9375 | 0.9538 | 0.9508 |
| 2 | 0.8891 | 0.9464 | 0.9459 | 0.9502 |
| 3 | 0.9027 | 0.8839 | 0.9581 | 0.9701 |
| 4 | 0.8982 | 0.9107 | 0.9556 | 0.9697 |
| 5 | 0.9163 | 0.8482 | 0.9669 | 0.9896 |

(ii) The 3-fold validation results are shown in the following Table 3.

[Table 3]

| | 3-fold | | | |
|---|---|---|---|---|
| average | 0.898 | 0.901 | 0.957 | 0.967 |
| SD | 0.006 | 0.024 | 0.007 | 0.007 |
| CV | 0.6% | 2.7% | 0.7% | 0.7% |

| concordance rate | | AUC | |
|---|---|---|---|
| for learning | for verification | for learning | for verification |
| 0.8913 | 0.9243 | 0.9502 | 0.9596 |
| 0.9022 | 0.8757 | 0.9637 | 0.9677 |
| 0.8995 | 0.9027 | 0.9562 | 0.9740 |

(iii) The 2-fold validation results are shown in the following Table 3.

[Table 4]

2-fold

| | | | | |
|---|---|---|---|---|
| average | 0.902 | 0.899 | 0.959 | 0.962 |
| SD | 0.005 | 0.015 | 0.009 | 0.015 |
| CV | 0.5% | 1.7% | 0.9% | 1.5% |

| concordance rate | | AUC | |
|---|---|---|---|
| for learning | for verification | for learning | for verification |
| 0.9055 | 0.8885 | 0.9650 | 0.9720 |
| 0.8989 | 0.9097 | 0.9527 | 0.9510 |

As understood from the concordance rate and AUC (at least 0.9 or above (i.e., generally high accuracy)), and standard deviation (SD) and coefficient of variation (CV) thereof in the above-mentioned Tables 2 to 4, as a result of validation 1, it was verified and confirmed that the setting (analysis) method of the formula (ii) of the present invention set in Example 2 is sufficiently appropriate and that extremely highly accurate differentiation is possible.

(2) Validation 2

From the results of the above-mentioned validation 1, it was sufficiently verified and confirmed that the validity of the formula (ii) (validity of analysis method) of the present invention set in Example 2. From the viewpoint of further increasing the reliability, the following validation 2 was performed.

In the method of validation 2, the raw data obtained by the above-mentioned clinical test (J Infect Chemother (2012) 18:832-840) was first divided into 2, a calculation formula (corresponding to formula (ii) of the present invention) was created for one of the 2 divisions, and the remaining 1 division was evaluated.

Then all the raw data were shuffled and divided into 2 again, a calculation formula (corresponding to formula (ii) of the present invention) was created for one of the 2 divisions, and the remaining 1 division was evaluated. This operation was repeated 5 times. The 5 evaluations were indicated as processings A, B, C, D, and E.

The results are shown in the following Table 5.

[Table 5]

processing A

| | | calculation formula | input value |
|---|---|---|---|
| | | −8.373 | |
| age (years old) | | −0.01673 | 1 |
| WBC (/μl) | | 5.56E−04 | 14000 |
| CRP (mg/dl) | | 0.8541 | 4 |

| bacterium | virus |
|---|---|
| 94.4% | 5.6% |

processing B

| | | calculation formula | input value |
|---|---|---|---|
| | | −8.468 | |
| age (years old) | | −9.96E−03 | 1 |
| WBC (/μl) | | 5.53E−04 | 14000 |
| CRP (mg/dl) | | 0.8795 | 4 |

| bacterium | virus |
|---|---|
| 94.2% | 5.8% |

processing C

| | | calculation formula | input value |
|---|---|---|---|
| | | −7.763 | |
| age (years old) | | −0.04979 | 1 |
| WBC (/μl) | | 5.23E−04 | 14000 |
| CRP (mg/dl) | | 0.7809 | 4 |

| bacterium | virus |
|---|---|
| 93.3% | 6.7% |

processing D

| | | calculation formula | input value |
|---|---|---|---|
| | | −8.468 | |
| age (years old) | | −9.96E−03 | 1 |
| WBC (μ/l) | | 5.53E−04 | 14000 |
| CRP (mg/dl) | | 0.8795 | 4 |

| bacterium | virus |
|---|---|
| 94.2% | 5.8% |

processing E

| | | calculation formula | input value |
|---|---|---|---|
| | | −8.512 | |
| age (years old) | | −0.03481 | 1 |
| WBC (/μl) | | 5.63E−04 | 14000 |
| CRP (mg/dl) | | 0.9007 | 4 |

| bacterium | virus |
|---|---|
| 95.0% | 5.0% |

As shown in Table 5, when the ratio indicating the possibility of bacterial infection and viral infection is used as an index, the ratios thereof are extremely close in all of the processings A to E. Therefore, also from the results of validation 2, it was verified and confirmed that the setting (analysis) method of the formula (ii) of the present invention set in Example 2 is sufficiently appropriate.

Example 4: Comparison of differentiation results of two relational expressions with different parameters

With respect to the blood samples collected from 286 cases of viral infection and 409 cases of bacterial infection (total 695 cases) with confirmed diagnosis by a PCR test, using multivariate analysis (multiple logistics regression) of StatFlex (Artec Co., Ltd.), regression coefficients ($a_0$ to $a_3$) of the above-mentioned formula (ii) were determined such that the area under the ROC curve (AUC) would reach the maximum in each case when viral infection and bacterial infection by PCR were selected as objective variables, and age, WBC, and CRP were selected as explanatory variables, and when age, neutrophil, and CRP were selected as explanatory variables. The results are shown in the following Tables 6-1 and 6-2.

[Table 6-1]

| order | variable name | β | SE(β) | z | P | odds ratio | 95% CI lower limit | 95% CI upper limit |
|---|---|---|---|---|---|---|---|---|
| 0 | | -5.733 | 0.4873 | | | | | |
| 1 | WBC | 3.147E-4 | 3.154E-5 | 9.979 | 0.00000 | 1.000 | 1.000 | 1.000 |
| 2 | CRP | 0.7162 | 0.06918 | 10.352 | 0.00000 | 2.047 | 1.787 | 2.344 |
| 3 | age | 0.1118 | 0.06156 | 1.815 | 0.06949 | 1.118 | 0.991 | 1.262 |
| AIC=449.145, AUC=0.950 | | | | | | | | |

[Table 6-2]

| order | variable name | β | SE(β) | z | P | odds ratio | 95% CI lower limit | 95% CI upper limit |
|---|---|---|---|---|---|---|---|---|
| 0 | | -4.218 | 0.3554 | | | | | |
| 1 | age | -0.09478 | 0.06679 | -1.419 | 0.15585 | 0.910 | 0.798 | 1.037 |
| 2 | neutrophil | 3.994E-4 | 3. 950E-5 | 10.112 | 0.00000 | 1.000 | 1.000 | 1.000 |
| 3 | CRP (1) | 0.7049 | 0.06944 | 10.151 | 0.00000 | 2.024 | 1.766 | 2.319 |
| AIC=438.792, AUC=0.949 | | | | | | | | |

The numerical values of each sample were respectively substituted into the formula (ii) having the regression coefficient shown in the above-mentioned Table 6-1 or 6-2, and the obtained numerical values (probability of bacterial infection) were plotted with the horizontal axis as explanatory variables (age+WBC+CRP) and the vertical axis as explanatory variables (age+neutrophil+CRP) for each confirmed diagnosis case of bacterial infection (left graph in Fig. 3) and confirmed diagnosis case of viral infection (right graph in Fig. 3). When each graph is divided into four parts, the lower left part and the upper right part show the cases where the differentiation results match between the two relational expressions, the part surrounded by the bold line matches the confirmed diagnosis, and the part surrounded by the dotted line indicates the cases that did not match the confirmed diagnosis. On the other hand, the upper left part and the lower right part of each graph show cases where the differentiation results did not match between the two relational expressions, and it was clarified that there are a certain number of cases that do not match. This indicates that erroneous judgment can be reduced by obtaining two differentiation results with two parameters as explanatory variables.

Example 5: Differentiation of severity of SARS-CoV-2 infection (COVID-19)

42 cases confirmed to be SARS-CoV-2 positive by PCR test were classified according to oxygen saturation (SPO2) into 31 severe cases (SPO2: less than 94) and 11 mild cases (SPO2: not less than 94). The blood samples collected from these cases and multivariate analysis (multiple logistics regression) of StatFlex (Artec Co., Ltd.) were used, severe COVID-19 and mild COVID-19 were selected as objective variables and WBC, CRP, platelet (PLT), lymphocyte (LYM) and the like were selected as explanatory variables, and it was evaluated and examined which combination would maximize the area under the ROC curve (AUC).

The results are shown in the following Tables 7-1 and 7-2.

[Table 7-1]

| order | variable name | β | SE(β) | z | P | odds ratio | 95% CI lower limit | 95% CI upper limit |
|---|---|---|---|---|---|---|---|---|
| 0 | | -4.520 | 1.695 | | | | | |
| 1 | WBC | -4.602E-4 | 3.699E-4 | -1.244 | 0.21346 | 1.000 | 0.999 | 1.000 |
| 2 | PLT | 0.1921 | 0.09938 | 1.933 | 0.05320 | 1.212 | 0.997 | 1.472 |
| 3 | CRP | 0.3317 | 0.1449 | 2.289 | 0.02206 | 1.393 | 1.049 | 1.851 |
| AIC=32.897, AUC=0.933 | | | | | | | | |

[Table 7-2]

| order | variable name | β | SE(β) | z | P | odds ratio | 95% CI lower limit | 95% CI upper limit |
|---|---|---|---|---|---|---|---|---|
| 0 | | -4.585 | 1.774 | | | | | |
| 1 | LYM | -6.455E-4 | 1.160E-3 | -0.557 | 0.57784 | 0.999 | 0.997 | 1.002 |
| 2 | PLT | 0.1420 | 0.07390 | 1.922 | 0.05459 | 1.153 | 0.997 | 1.332 |
| 3 | CRP | 0.1904 | 0.09928 | 1.918 | 0.05510 | 1.210 | 0.996 | 1.470 |
| AIC=34.285, AUC=0.903 | | | | | | | | |

A similar high AUC was obtained when the CRP value, WBC count, and PLT count were used as explanatory variables, and when the CRP value, LYM count, and PLT count were used as explanatory variables.

Using the relational formula (ii) having the regression coefficient shown in the above-mentioned Table 7-1 or 7-2, cases of severity differentiation by substituting actual test values are shown in Tables 8-1 and 8-2.

[Table 8-1]

| | calculation formula | input value | |
|---|---|---|---|
| | −4.52 | | |
| WBC | −0.0004602 | 8000 | /μL |
| PLT | 0.1921 | 300000 | /μL |
| CRP | 0.3317 | 1 | mg/dL |

| severe | mild |
|---|---|
| 10.8% | 89.2% |

| | calculation formula | input value | |
|---|---|---|---|
| | −4.52 | | |
| WBC | −0.0004602 | 5000 | /μL |
| PLT | 0.1921 | 300000 | /μL |
| CRP | 0.3317 | 10 | mg/dL |

| severe | mild |
|---|---|
| 90.5% | 9.5% |

[Table 8-2]

|  | calculation formula | input value |  |
|---|---|---|---|
|  | −4.519 |  |  |
| lymphocyte | −0.001282 | 1000 | /μL |
| PLT | 0.1369 | 300000 | /μL |
| CRP | 0.2276 | 1 | mg/dL |

| severe | mild |
|---|---|
| 18.7% | 81.3% |

|  | calculation formula | input value |  |
|---|---|---|---|
|  | −4.519 |  |  |
| lymphocyte | −0.001282 | 500 | /μL |
| PLT | 0.1369 | 300000 | /μL |
| CRP | 0.2276 | 10 | mg/dL |

| severe | mild |
|---|---|
| 77.3% | 22.7% |

[Industrial Applicability]

According to the present invention, determination information useful for a physician in differentiating whether a patient is suffering from a viral infection or a bacterial infection can be provided. As a result, misprescriptions such as using antibiotics for a viral cold are suppressed, and problems caused by the misprescriptions, such as the emergence of resistant bacteria, are suppressed.

According to the present invention, moreover, it has become possible to objectively determine whether a patient with a viral infection (e.g., COVID-19) is severely ill, without depending on the subjective judgment of the patient him/herself or an interviewer, and the necessity of therapeutic intervention, and selection of treatment methods can be appropriately determined.

This application is based on a patent application No. 2020-113607 filed in Japan (filing date: June 30, 2020), the contents of which are incorporated in full herein by reference.

[Explanation of symbols]

| | |
|---|---|
| M1 | blood sample |
| 10 | CRP measuring chamber |
| 20 | red blood cell measuring chamber |
| 30 | white blood cell measuring chamber |
| 100 | blood measuring mechanism |
| 200 | control part |
| 220 | analysis part |
| 300 | mechanism control part |
| 400 | measured value receiving part |
| 500 | measured value processing part |
| 510 | CRP value calculating part |
| 520 | red blood cell count calculating part |
| 530 | white blood cell count calculating part |
| 600 | parameter receiving part |
| 700 | infection determining part |
| 710 | relational expression holding part |

800    calculation result output part

**Claims**

1.  A body fluid analysis device, comprising

    a parameter receiving part that receives plural kinds of parameters for use in the determination of whether a test subject is suffering from a viral infection or a bacterial infection, wherein said plural kinds of parameters comprise a CRP value of a body fluid sample collected from the test subject, and test value(s) of one or more body fluid test items selected from body fluid test items other than the CRP value, and
    an infection determining part configured to calculate determination information of whether the test subject is suffering from a viral infection or a bacterial infection based on said plural kinds of parameters and using a relational expression (I) described below and stored in advance, wherein said relational expression (I) is a formula showing a relationship between explanatory variables and an objective variable as determined by a multivariate analysis,

        wherein the explanatory variables are the same plural kinds of parameters as said plural kinds of parameters and obtained from a plurality of data providers known to have suffered from a viral infection and a plurality of data providers known to have suffered from a bacterial infection, and
        the objective variable is determination information as to whether each of said plurality of data providers suffered from a viral infection or a bacterial infection.

2.  The body fluid analysis device according to claim 1, wherein the multivariate analysis comprises one or more analyses selected from a discriminant analysis, a logistic regression analysis, a multiple regression analysis, a Cox hazard regression analysis, a principal component analysis, a factor analysis, a variance analysis, and a cluster analysis.

3.  The body fluid analysis device according to claim 1 or 2, wherein the test value(s) of the body fluid sample relating to the one or more body fluid test items selected from body fluid test items other than the CRP value comprises a white blood cell count, and the body fluid analysis device further comprise(s) a white blood cell measuring chamber and a white blood cell count calculating part for measuring the white blood cell count of the body fluid sample collected from the test subject, and a CRP measuring chamber and a CRP value calculating part for measuring the CRP value of the body fluid sample.

4.  The body fluid analysis device according to any one of claims 1 to 3, wherein the plural kinds of parameters for use in the determination of whether the test subject is suffering from a viral infection or a bacterial infection comprise a neutrophil count, and the infection determining part is configured to calculate determination information showing a possibility of the test subject suffering from a viral infection or a bacterial infection based on at least a white blood cell count and the CRP value and/or at least a neutrophil count and the CRP value included in the test values, and using the relational expression (I).

5.  The body fluid analysis device according to any one of claims 1 to 4, further comprising a chamber and a neutrophil count calculating part for measuring a neutrophil count of the body fluid sample collected from the test subject.

6.  The body fluid analysis device according to any one of claims 1 to 5, wherein the body fluid is blood.

7.  A method for determining whether a body fluid sample derived from a test subject is obtained from a test subject with a viral infection or a test subject with a bacterial infection, comprising

    a first step of preparing plural kinds of parameters (A) below, and
    a second step of determining whether the body fluid sample is obtained from a test subject with a viral infection or a test subject with a bacterial infection by using the plural kinds of parameters and a relational expression (I) described below, wherein the plural kinds of parameters (A) comprise a CRP value of the body fluid sample and test value(s) of one or more body fluid test items selected from body fluid test items other than the CRP value, and wherein the relational expression (I) is a formula showing a relationship between explanatory variables and an objective variable as determined by a multivariate analysis,

        wherein the explanatory variables are the same plural kinds of parameters as said plural kinds of parameters and obtained from a plurality of data providers known to have suffered from a viral infection and a plurality of data providers known to have suffered from a bacterial infection, and

the objective variable is determination information as to whether each of said plurality of data providers suffered from a viral infection or a bacterial infection.

8. The method according to claim 7, wherein the test value(s) of the body fluid sample relating to the one or more body fluid test items selected from body fluid test items other than the CRP value in the plural kinds of parameters (A) comprises a white blood cell count.

9. The method according to claim 7 or 8, wherein the body fluid is blood.

10. A computer program for functioning a computer as a parameter receiving means that receives plural kinds of parameters of the following (A) for use in the determination of whether a test subject is suffering from a viral infection or a bacterial infection, and as an infection determining means that calculates determination information of whether the test subject is suffering from a viral infection or a bacterial infection based on said plural kinds of parameters and using the following relational expression (I),

wherein the plural kinds of parameters of (A) comprise a CRP value of said body fluid sample derived from the test subject and test value(s) of one or more body fluid test items selected from body fluid test items other than the CRP value, and wherein the relational expression (I) is a formula showing a relationship between explanatory variables and an objective variable as determined by a multivariate analysis,

wherein the explanatory variables are the same plural kinds of parameters as said plural kinds of parameters and obtained from a plurality of data providers known to have suffered from a viral infection and a plurality of data providers known to have suffered from a bacterial infection, and

the objective variable is determination information as to whether each of said plurality of data providers suffered from a viral infection or a bacterial infection.

11. The computer program according to claim 10, wherein the test value(s) of the body fluid sample relating to the one or more body fluid test items selected from body fluid test items other than the CRP value in the plural kinds of parameters (A) comprises a white blood cell count.

[Fig. 1]

EP 4 152 000 A1

[Fig. 2]

start

| s1: sample liquid for CRP measurement is formed in CRP chamber | s5: sample liquid for white blood cell counting is formed in WBC chamber |

s2: measured value corresponding to CRP value is obtained in CRP chamber

s6: white blood cells are counted in WBC chamber

s3: measured value receiving part receives CRP parameter value

s7: measured value receiving part receives measured value of white blood cell counting

s4: CRP value calculating part determines CRP value

s8: white blood cell count calculating part calculates white blood cell count

s9: infection determining part substitutes CRP value and white blood cell count into relational expression (I) stored as explanatory variables relating to test subject and calculates objective variable Y and discriminant information of whether viral infection or bacterial infection

end

[Fig. 3]

## INTERNATIONAL SEARCH REPORT

| | International application No. |
|---|---|
| | PCT/JP2021/024579 |

### A. CLASSIFICATION OF SUBJECT MATTER
Int.Cl. G01N33/49(2006.01)i, G01N33/50(2006.01)i, G01N33/68(2006.01)i
FI: G01N33/49Z, G01N33/68, G01N33/50K

According to International Patent Classification (IPC) or to both national classification and IPC

### B. FIELDS SEARCHED

Minimum documentation searched (classification system followed by classification symbols)
Int.Cl. G01N33/49, G01N33/50, G01N33/68

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2015-510122 A (MEMED DIAGNOSTICS LTD.) 02 April 2015 (2015-04-02), claims 7-10, 15, 18-26, paragraphs [0152]-[0156], [0182], [0250]-[0272], [0283]-[0374] | 1-11 |
| X | JP 2017-532633 A (MEMED DIAGNOSTICS LTD.) 02 November 2017 (2017-11-02), claims, paragraphs [0252]-[0328], [0378]-[0586] | 1-11 |
| X | JP 2017-536361 A (KONINKLIJKE PHILIPS N.V.) 07 December 2017 (2017-12-07), claims, paragraphs [0145]-[0148], [0268]-[0297], [0364]-[0368] | 1-11 |
| X | US 2019/0011456 A1 (MEMED DIAGNOSTICS LTD.) 10 January 2019 (2019-01-10), claims, drawings, paragraphs [0334]-[0521] | 1-11 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 August 2021 | 14 September 2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

International application No.

PCT/JP2021/024579

**INTERNATIONAL SEARCH REPORT**

C (Continuation).   DOCUMENTS CONSIDERED TO BE RELEVANT

| Category* | Citation of document, with indication, where appropriate, of the relevant passages | Relevant to claim No. |
|---|---|---|
| A | 細菌感染時の CRP と WBC, HORIBA News Letter, 2010, no. 2, https://www.horiba.com/fileadmin/uploads/Medical-Diagnostics/Documents/tec_jp/3uhw_hnl_no2.pdf, entire text, non-official translation (CRP and WBC during bacterial infection) | 1-11 |

Form PCT/ISA/210 (second sheet) (January 2015)

INTERNATIONAL SEARCH REPORT
Information on patent family members

International application No.

PCT/JP2021/024579

| | | | |
|---|---|---|---|
| JP 2015-510122 A | 02 April 2015 | WO 2013/117746 A1<br>claims 7-10, 15, 18-26,<br>paragraphs [000191]-[000194], [000221],<br>[000305]-[000327], [000339]-[000420]<br>US 2015/0017630 A1<br>CN 104204803 A | |
| JP 2017-532633 A | 02 November 2017 | US 2017/0235871 A1<br>claims, paragraphs [0148]-[0214],<br>[0303]-[0379], [0429]-[0673]<br>WO 2016/024278 A1<br>KR 10-2017-0041907 A<br>CN 107076746 A | |
| JP 2017-536361 A | 07 December 2017 | US 2017/0356921 A1<br>claims, paragraphs [0229]-[0232],<br>[0363]-[0405], [0501]-[0503]<br>WO 2016/079219 A1<br>CN 107001455 A | |
| US 2019/0011456 A1 | 10 January 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 11108923 A
- JP 2014215210 A
- JP 2020113607 A

### Non-patent literature cited in the description

- **TAKAFUMI OKADA et al.** A practical approach estimating etiologic agents using real-time PCR in pediatric inpatients with community-acquired pneumonia. *J Infect Chemother,* 2012, vol. 18, 832-840

- *J Infect Chemother,* 2012, vol. 18, 832-840